# EUROPEAN PATENT SPECIFICATION

(11) **EP 3 659 563 B1**
(45) Date of publication and mention of the grant of the patent: **04.05.2022**
(21) Application number: 18209119.9
(22) Date of filing: 29.11.2018
(51) Int. Cl.: A61F 13/49

(54) **ELASTICISED ABSORBENT ARTICLE**
ELASTIFIZIERTER SAUGFÄHIGER ARTIKEL
ARTICLE ABSORBANT ÉLASTIQUE

(43) Date of publication of application: 03.06.2020
(73) Proprietor: Ontex BV, 9255 Buggenhout (BE); Ontex Group NV, 9320 Erembodegem (BE)
(72) Inventor: WEBER, Ainas, 53474 Bad Neuenahr-Ahrweiler (DE); VERTONGEN, Kjenta, 9255 Buggenhout (BE); DHOLLANDER, Tara, 9255 Buggenhout (BE); VANDE PUTTE, Els, 9255 Buggenhout (BE); RASSCHAERT, Romy, 9255 Buggenhout (VE)
(74) Representative: Larangé, Françoise

(56) References cited:
- WO-A1-2016/175780
- US-A1- 2008 287 898
- US-A1- 2017 079 849
- US-B2- 8 007 892

## Description

### TECHNICAL FIELD

The invention pertains to the technical field of absorbent hygiene products. In particular, the present invention relates to an absorbent article for absorbing body fluids and exudates, such as urine and fecal material. More particularly, the present invention relates to disposable and refastenable absorbent garments, such as disposable two-in-one pant-diapers, which are configured to collect and contain fecal material and avoid leakage.

### BACKGROUND

Absorbent articles having defined core and chassis regions are supposed to have a comfortable fit about the wearer. Traditionally re-fastenable diapers have been used due to their versatility and ease of fitting onto the wearer, especially when the wearer is laying down.

EP 1 350 493 A1 discloses an absorbent product having a first end portion with two side flaps extending laterally outwardly on either side of a waist section. This disclosure exemplifies embodiments of traditional diapers.

Current traditional diaper design frequently includes the use of back ears. Back ears may extend laterally from the longitudinal edge of the rear waist section of the chassis. The engaging member of the fastening system may be attached to the back ear. When the fastening system is engaged, the back ear serves as an interconnecting member between the front waist section and the rear waist section, which together form a waist opening and pair of leg openings. Common back ear construction involves a polymeric material laminated between two substrates. Often back ear construction involves an elastomeric material laminated between two substrates that are supple, soft, and non-irritating to a wearer's skin such as a nonwoven material. Elastomeric films are commonly used since the film provides a degree of stretch to the waist circumference. This stretch allows the diaper to provide a more customized fit. Furthermore, the stretch capability allows the diaper to adjust to the forces exerted by the wearer without causing permanent deformation of the diaper or discomfort for the wearer of the diaper.

More recently, however, the trend has shifted towards the use of pant diapers due to the ease and comfort of being able to pull the pant up and down over the hips, being particularly suitable for babies, toddlers, children and adults that can crawl and/or stand. It is known to make such absorbent pants with elasticized stretchable side portions and waist portion, usually comprising elastic members, such as elastic threads, contractably affixed between the backsheet and the topsheet.

It is further known to make portions of the chassis of absorbent articles of an elastic material, such as stretch-bonded laminates. Such laminates may include a layer of meltblown elastomeric fibers which have been stretched and sandwiched between outer layers of spunbonded webs.

U.S. Pat. No. 6,552,245 discloses an extensible outer cover for an absorbent article which provides a certain permanent deformation when subjected to a tensile force. The extensible outer cover comprises a necked laminate in the form of one layer of a necked non-elastic film and one layer of an elastic film. The films may be breathable.

WO 03/047488 discloses an elastic laminate comprising an elastic film which on opposite sides is bonded to first and second non-elastic fibrous layers. The laminate is made by bonding the non-elastic fibrous layers to the elastic film layer and subsequently stretching the composite material, causing the non-elastic materials to break. The elastic film material may be of a breathable material. The laminate may be incorporated in an absorbent article.

US 2004/0243086 discloses a disposable pant-like undergarment having stretchable front and back portions, for example made of an elastic laminate. An absorbent assembly is secured to the front and back portions.

Further examples of absorbent articles which in part are made of elastic laminates are found in U.S. Pat. No. 6,476,289 and JP 10043235.

Further efforts have been made in order to improve the efficiency of production and assembly of pant diapers and several common in-line processes are exemplified in WO200430477A1, US2008/0287898A1, EP 1 602 348 A1, and EP 1 240 881 A2.

Although all above described variants of pants provide benefits over traditional diapers especially in terms of fit and comfort, checking the status of the wearer's absorbent garment contents can be just as cumbersome as changing the absorbent garment. Moreover, fitting the pants when the wearer is not able to stand (i.e. when laying flat) is also more cumbersome compared to traditional diapers.

Thus, traditional diapers and pants each have their advantages and disadvantages and a need exists for a single product capable of combining all the advantages of both. Some attempts have been made in order to combine both solutions into a single product, these are exemplified in US9,615,980B2, US2016/0374872A1, and US2004/0102745A1. However, such refastenable pant constructions introduce cost and production complexities with the overall fit being compromised, especially when compared with classic pants.

In an attempt to overcome the latter drawbacks, WO02/069866A1 describes a refastenable pant-diaper that is tuckable in a pre-fastened state wherein the side panels are folded in a particular way to ensure that the hooks of the mechanical fasteners face away from the wearer's skin. This arrangement introduces advantages such as reduced skin irritation and perceived comfort, however still has disadvantages particularly on production complexity and cost as well as fit over extended periods of time.

US 2017/0079849 discloses a taped absorbent article comprising a discrete chassis. A discrete belt portion is joined to a portion of the discrete chassis, said discrete belt portion comprising three zones each comprising at least one of the plurality of elastic members. Panels taught in US 2017/0079849 may comprise a back panel being an elastic laminate (nonwoven layers joined to elastic layers) in combination with a front panel that may or may not be elastic. In such embodiment, rectangular panels are taught. However articles according to US 2017/0079849 still can be improved, for example to improve the fit over extended periods of time whilst allowing simple and cost-effective manufacture.

A need therefore exists for a hybrid disposable diaper-pant that truly provides the benefits of a two-in-one product in relation to comfort, fit, and overall usability in a cost effective manner.

### SUMMARY OF THE INVENTION

In a first aspect, the present disclosure relates to an absorbent article, preferably in the form of a pre-fastened two-in-one diaper-pant, comprising: one or more discrete front panels, preferably in the front section of the article; a discrete back panel, preferably in the back section of the article; and an absorbent insert joined to the front and back panels wherein said front and back panels are separated from each other along a longitudinal axis y-y running parallel to a length of said insert and crossing both said front and back panels and wherein said insert is interposed between said front panel and said back panel such to form a substantially H-shaped absorbent article (typically said shape being viewed when the article is in a laid-flat state), wherein the front and back panels have a total width **W** being greater than a width **w** of said insert along a transverse axis **x-x,** and wherein said insert comprises a liquid permeable topsheet, a liquid impermeable backsheet, and an absorbent material sandwiched between said topsheet and backsheet, wherein the back panel consists of a unitary panel and wherein the front and/or back panels comprise one or more fasteners such that the front panel and back panel can be refastenably coupled together to a closed position onto a wearer, and wherein only one of the front panel and the back panel is elastic, and wherein the unitary back panel (3) has a substantially trapezoidal shape.

In a second aspect, the present disclosure relates to a process of making an absorbent article comprising the, preferably sequential, steps of: providing a first, preferably inelastic, web having a width extending perpendicular to a machine direction **MD** and a length extending parallel to said machine direction **MD** and accelerating said first web along said machine direction **MD;** severing the first web along cutting lines extending along the entire width thereof, said cutting lines being sequentially applied along said machine direction **MD** and wherein at least each second cutting line in a sequence of cutting lines run substantially parallel to each other, such that a plurality of discrete front panels are formed; providing a second, preferably elastic, web having a width extending perpendicular to a machine direction **MD** and a length extending parallel to said machine direction **MD** and accelerating said second web along said machine direction **MD;** severing the second web along cutting lines extending along the entire width thereof, said cutting lines being sequentially applied along said machine direction **MD** and wherein at least each second cutting line in a sequence of cutting lines run substantially parallel to each other, such that a plurality of discrete back panels are formed; providing a plurality of inserts comprising a liquid permeable topsheet, a liquid impermeable backsheet and an absorbent material sandwiched therebetween; joining only one of said back panels to each said inserts at a first end thereof; joining one or two, preferably only one, of said front panels to each said inserts at a second end thereof being opposite said first end and distanced along the longitudinal axis **y-y;** applying one or more fasteners to the front and/or back panels; and wherein only one of the front panel and the back panel is elastic.

### DESCRIPTION OF FIGURES

**Fig. 1** is a schematic plan view of an unassembled and fully stretched absorbent article according to an embodiment of the present disclosure.
**Fig. 2** is a schematic plan view of an unassembled and fully stretched absorbent article according to an embodiment of the present disclosure.

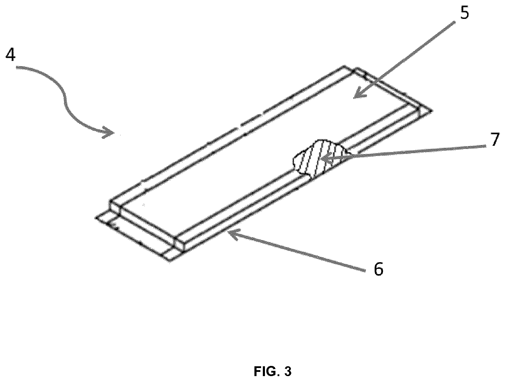

**Fig. 3** is an isometric view of an insert according to an embodiment of the present disclosure.

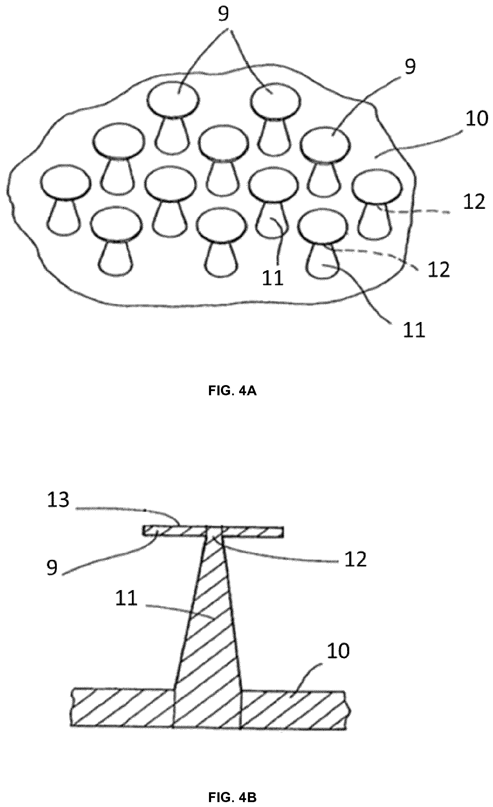

**Fig. 4A and 4B** are schematics of a fiber fastener according to an embodiment of the disclosure. With Fig.4A showing a plurality of fiber fasteners on a patch or tape and Fig.4B an exemplary individual fiber fastener.
**Fig. 5** is a schematic illustrating the attachment region, or joining area, of the insert to the panels according to an embodiment of the disclosure.
**Fig. 6** is a schematic illustrating the attachment region, or joining area, of the insert to the panels according to an embodiment of the disclosure.
**Fig. 7** is a schematic illustrating the attachment region, or joining area, of the insert to the panels according to an embodiment of the disclosure.
**Fig. 8A and 8B** are schematics illustrating the process of making an absorbent article according to an embodiment of the present disclosure.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

An incontinence absorbent article, such as a diaper, comprises a front waistband region, a back waistband region, an intermediate crotch region which interconnects the front and rear waistband regions. When used herein, reference to a "front" portion refers to that part of the diaper which is generally located on the front of an incontinent person when in use. Reference to the "rear" portion refers to the portion of the diaper generally located at the rear of the incontinent person when in use, and reference to the "crotch" portion refers to that portion which is generally located between the legs of an incontinent person when in use. The crotch region is an area where repeated fluid surge typically occurs.

Preferably, a diaper comprises a liquid permeable "top sheet", a liquid impermeable "back sheet", and an "absorbent medium" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sub-layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

"Activatably breathable" as used herein means that the element referred to becomes breathable only after activation for example by the user when applying the absorbent article to a wearer. This term may exclude materials that are made breathable during manufacture and rather generally covers materials that are non-breathable after manufacture of the article but that become breathable when warn by a subject.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

The terms "back section" and "rear back section" are used herein as synonyms and refer to the area of the absorbent article which is contact with the back of the wearer when the absorbent article is worn.

The term "back sheet or backsheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "belly section" or "front section" and "front belly section" are used herein as synonyms and refer to the area of the absorbent article which is contact with the belly of the wearer when the absorbent article is worn.

As used herein, the "body-facing" or "bodyside" or "skin facing" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m² - 24 hours.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.

"Hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "density" or "concentration" when referring to the absorbent material, in particular the SAP, of a layer, refers to the amount of the absorbent material divided by the surface area of the layer over which the absorbent material is spread out.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "elastic resistance" describes an elastic force that tends to resist an applied tensile force causing a material provided therewith to tend to contract to an untensioned configuration in response to a stretching force.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction typically at least parallel to the transverse axis by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent). "Extension" means the change in length of a material due to stretching (expressed in units of length).

As used herein the term "extensible" means elongatable in at least one direction, but not necessarily recoverable.

The term "finished" or "final", when used with reference to a product, means that the product has been suitably manufactured for its intended purpose.

The term "flexible" refers to materials which are compliant and which will readily conform to the general shape and contours of the wearer's body.

As used herein, the term "garment" means any type of apparel which may be worn. This includes diapers, training pants, incontinence products, surgical gowns, industrial workwear and coveralls, undergarments, pants, shirts, jackets and the like.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another.

"Pre-fastened two-in-one diaper-pant" as used herein means a refastenable diaper that in its final form is a pant that comprises a reclosable seam.

"Pre-fastenable two-in-one diaper-pant" as used herein means a refastenable diaper that is adapted and/or capable of being arranged as a pant that comprises a reclosable seam in its final form.

"Refastenable" as used herein means an article that can be closed (i.e. fastened), opened (i.e. de-fastened) and re-closed (i.e. refastened) a plurality of times generally with a closing force that stays substantially constant between each iteration of opening and closing, and typically without any portion of the article being torn or damaged.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

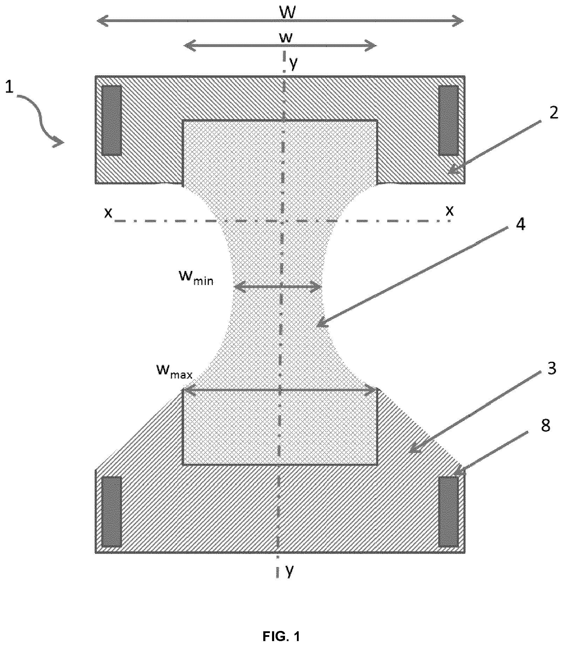

The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer (i.e. typically in a manner as illustrated in Fig.1 to Fig.2).

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

"Longitudinal" is a direction running parallel to the maximum linear dimension of the article.

The term "nonelastic" or "inelastic" refers to any material which does not fall within the definition of "elastic" above, typically by not comprising elastic materials such as elastic web materials described herein.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

"Pulp fluff" or "fluff pulp" refers to a material made up of cellulose fibers. The fibers can be either natural or synthetic, or a combination thereof. The material is typically lightweight and has absorbent properties.

As used herein the term "sheet" or "sheet material" refers to woven materials, nonwoven webs, polymeric films, polymeric scrim-like materials, and polymeric foam sheeting.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. "Stretch" may be unidirectional, bidirectional, or multi- directional. The specific "stretch" properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

By "unitary", it is meant that the element referred to forms a single or uniform body. Preferably wherein the term excludes laminates comprising the combination of films and nonwovens.

Use of the term "substrate" includes, but is not limited to, woven or nonwoven webs, porous films, ink permeable films, paper, composite structures, or the like.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "top sheet or topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

Embodiments of the articles according to the disclosure will now be described.

### THE ABSORBENT ARTICLE

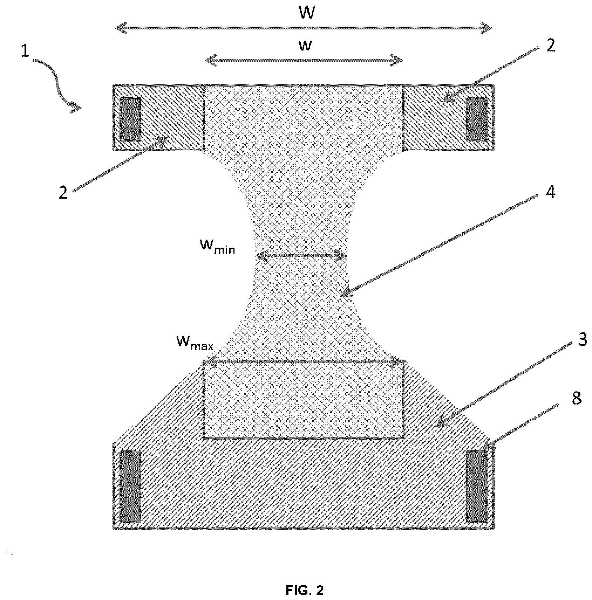

In a first aspect and with reference to Figs. 1 and 2, the disclosure herein relates to an absorbent article **1,** preferably in the form of a pre-fastenable two-in-one diaper-pant, comprising: one or more discrete front panels **2,** preferably in the front section of the article; a discrete back panel **3,** preferably in the back section of the article; and an absorbent insert **4** joined to the front and back panels **2,3,** wherein said front and back panels **2,3** are separated from each other along a longitudinal axis **y-y** running parallel to a length of said insert **4** and crossing both said front and back panels **2,3,** and wherein said insert **4** is interposed between said front panel **2** and said back panel **3** such to form a substantially H-shaped absorbent article (typically said shape being viewed when the article is in a laid-flat state), wherein the front and back panels **2,3** have a total width **W** being greater than a width **w** of said insert **4** along a transverse axis **x-x,** and wherein said insert **4** comprises a liquid permeable topsheet **5,** a liquid impermeable backsheet **6,** and an absorbent material **7** sandwiched between said topsheet **5** and backsheet **6,** wherein the back panel **3** consists of a unitary panel and wherein the front and/or back panels (2,3) comprise one or more fasteners (8) such that the front panel **2** and back panel **4** can be refastenably coupled together to a closed position onto a wearer, and wherein only one of the front panel **2** and the back panel **3** is elastic, and wherein the unitary back panel (3) has a substantially trapezoidal shape and preferably wherein both front and back panels **2,3** are liquid permeable. Advantageously, this allows for optimal slip-on of the article around the wearer's torso as well as providing a tighter and snug fit resulting from the joining of the elastic surface directly to the inelastic surface. A single or unitary elastic panel further allows to retain fit over extended periods of time (provided by the interaction of inelastic portions of the article, such at least one of the panels, and the larger substantially uninterrupted surface of the elastic panel) which can be manufactured in a simple and cost-effective way. Typically, the front and back panels **2,3** herein are not directly joined together but are rather indirectly connected together via the insert **4.**

As exemplified in Fig. 1, the front panel **2** is preferably a single or unitary panel typically such that continuous front and back belts are formed onto which the insert is directly joined allowing for improved overall comfort of the article. Alternatively, as shown in Fig.2, the absorbent article may comprise two oppositely disposed panels **2** along the transverse axis **x-x,** although this embodiment may reduce the amount of raw material used it is less preferred compared to the previously described embodiment since comfort in the belly portion of the wearer is impacted.

Preferably, the front panel **2** is inelastic and the back panel **3** is elastic, typically wherein the front panel **2** is composed entirely of one or more inelastic nonwoven layers, and wherein the back panel **3** consists of a laminate comprising an elastic film layer, or a plurality of elastic strands, and one or more nonwoven layers; or consists of one or more elastic nonwoven layers, preferably consists of a unitary elastic nonwoven layer.

In an embodiment, the insert **4** is joined to the front and back panels **2,3** at discrete joining areas **14,** wherein said discrete joining areas are less than 20%, preferably less than 15%, more preferably from 2% to 10%, even more preferably from 4% to 8%, of the total surface area of said insert **4** taken from a plane parallel to that formed by the longitudinal axis y-y and transverse axis **x-x** (i.e. when viewed from a laid-flat fully extended state). Preferably, the discrete joining areas **14** are present at positions, preferably at only two positions, proximal to the front and back of the absorbent articles and at no position therebetween. An advantage of this embodiment is that the stretch properties of the panel are not negated and unnecessary stiffening is avoided in order to provide a snug and comfortable fit with limited wrinkles being formed, and this for a number of belly circumferences of the target subject wearing the article.

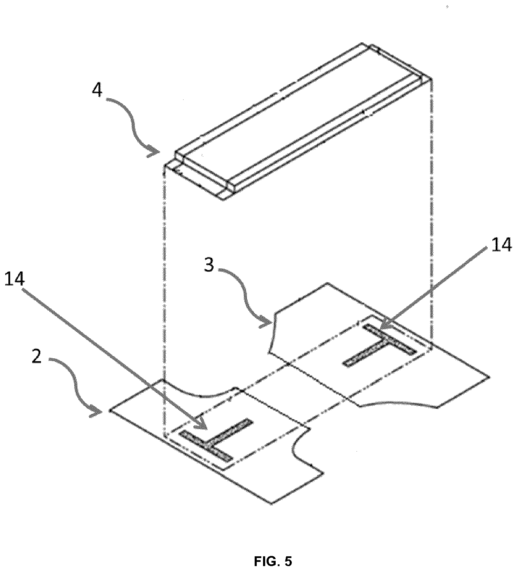

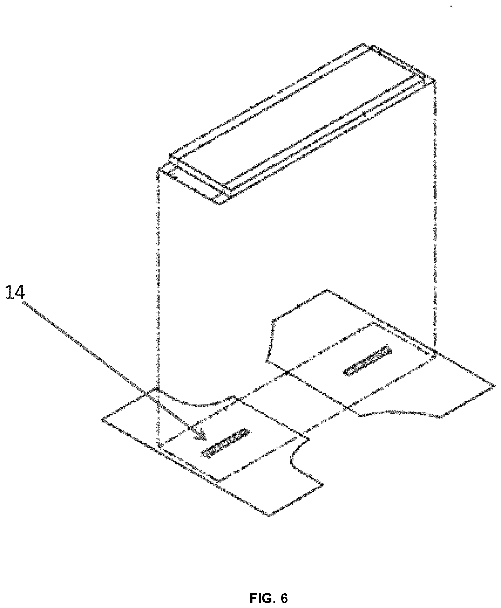

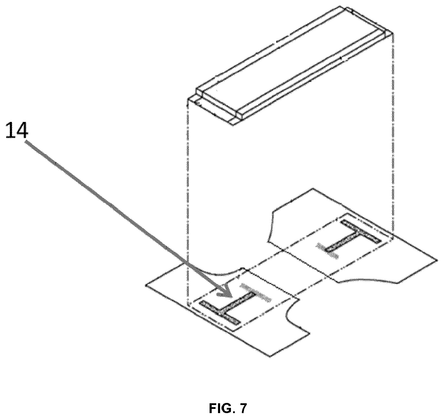

As shown in Figs. 5-7, the discrete joining areas **14** may have a shape selected from the group consisting of line-form, cross-form, X-shaped, Y-shaped, H-shaped, and T-shaped, preferably line-form, more preferably said joining areas comprising an adhesive. Advantageously this arrangement allows for optimal anchoring whilst limiting stiffening effects that rather would impact comfort.

In an embodiment, the joining areas **14** comprise an adhesive, preferably a hotmelt adhesive. Alternatively, the insert **4** is joined to the panels **2,3** at the discrete joining areas **14** via mechanical bonding typically selected from the group consisting of ultrasonic bonding, thermal pressure bonding, and pressure bonding.

In a preferred embodiment, the insert **4** is hourglass shaped and comprises a minimum width **Wₘᵢₙ** and a maximum width **Wₘₐₓ** wherein the ratio **Wₘᵢₙ/Wₘₐₓ** is from 0.40 to 0.80, preferably from 0.45 to 0.75, more preferably from 0.50 to 0.65. Advantageously it has been found that such shape in combination with a single or unitary elastic back panel provides for added comfort. If the ratio is too low insufficient coverage and absorbtion in the crotch region is attained, whilst if too high creases and or wrinkles are formed between the legs of the wearer due to the added elastification created by the panel which results in discomfort and higher risk of unwanted leakages.

The front and/or back panels **2,3** comprise one or more fasteners **8** generally arranged to provide a refastenable closure between said front, back panels **2,3,** and/or garment facing surface of the insert **4.** The fasteners may be directly applied onto a surface of said panels **2,3** or may be indirectly applied thereto via one or more tape tabs. Preferably the fasteners **8** are in the form of adhesive and/or mechanical fasteners, and may comprise, or consist of, mechanical fasteners in the form of fiber fasteners. In a preferred embodiment, the back **3** panel comprises mechanical fiber fasteners on the skin facing side thereof and arranged to join to a nonwoven garment facing surface of the front panel **2** and/or insert **4.**

Preferably, the fiber fastener, when used, comprises a plurality of bonding elements spaced apart from one another, said bonding elements each comprising a head part **9,** a backing part **10** and a stem part **11** connecting the head part **9** and the backing part **10,** preferably integrally connected together, and extending therebetween, and wherein the head part **9** consists of a head plate, the diameter of which may be greater than the diameter on every point of the stem part **11** that is conical in form, wherein the stem part **11** is connected flexibly to the head plate by means of an articulation part **12,** and that a contact surface **13** of the head part **9** enables detachable adhesion between the front panel **2,** back panel **3** and/or garment facing surface of the insert **4.** Advantageously, this arrangement allows for better joining of the substrates compared to known hook and loop (or Velcro^{®}) type fasteners. Indeed, this particular arrangement with the particular geometry described and articulation part has been found beneficial for joining onto any nonwoven and/or film-based counterpart (or counterjoining) surface thus allowing a larger range of closing positions without having to coat/cover a large portion of the counterpart surface with loop material that rather adds cost and increases chances of soiling and reduced effectiveness. This has been found to synergistically work particularly well when used in combination with a single or unitary elastic back panel to form the pre-fastened two-in one diaper pants herein.

"Fiber fasteners" as used herein generally means fasteners wherein the maximum diameter of the stem part is less than 50 µm, preferably from 10 µm to 40 µm, more preferably from 15 µm to 30 µm, and typically wherein the minimum diameter of the stem part **11** is less than 30 µm, preferably from 1 µm to 15 µm, more preferably from 5 µm to 9 µm, and wherein the ratio of the perimeter of the contact surface **13** to the maximum diameter of the stem may be from 0.5 to 2, preferably from 0.8 to 1.5, more preferably from 0.9 to 1.4. Commercially available fiber fasteners suitable for use herein comprise the micro-structured silicone fastener film manufactured and supplied by Gottlieb Binder GmbH u. Co KG, for example under the Gecko^{®} brand such as Gecko^{®} Nanoplast^{®} tape.

In an embodiment, the head part **9** is in the form of a plate and has essentially a uniform thickness or, as viewed in cross-section, has a wedge or double wedge shape, and that its outer periphery follows a curved, in particular circular path or is polygonal, in particular hexagonal in shape. Such arrangement allows for the most optimal surface area for releasable attachement. Preferably, detachment of the head part **9** takes the form of a peeling motion from a counterjoining surface when the stem **11** is tilted around the articulation part **12** by an angle of at least 20°, preferably of at least 40°, relative to a vertical axis extending perpendicular to a plane of the head part **9.** Typically, the fiber fastener is made at least partially of a flexible plastic material which can be processed by means of a micro-replication process.

Preferably, the fiber fasteners, or at least the contact surface **13** of the head part **9** of the fiber fasteners, comprises a silicon-based material, more preferably is made of polyvinyl siloxane, preferably the entire fastener is made of said material. The rubber-like properties of such materials when cured allow for creating flexible surfaces for attachment to a variety of film and nonwoven counterpart surfaces via Van der Waals forces of attraction. Moreover, it has advantages over standard hook & loop wherein said fiber fasteners made of such materials limit scratching and skin irritation in case of contact and rubbing.

In an embodiment, the stem part **11** is off-centred to the head part **9.** This arrangement allows for a non-symmetrical cantilevering that enables improved adhesion resistance to the counterpart surfaces to be joined. Preferably, the stem part **11** is connected to the head part **9** at different positions for each directly neighboring fiber fasteners in a series of said fasteners generally within each patch or tape comprising a plurality of said fiber fasteners. Preferably, the tapered stem part **11** is coupled by means of an articulation part **13** to a head part **9** in the form of a head plate which runs substantially flat.

In a preferred embodiment, the back panel is activatably breathable, preferably wherein the laminate comprises a fluid impervious film layer having a first side and a second side; a first non-woven layer spot bonded at a plurality of weld points to said first side of said film layer; a second non-woven layer spot bonded at a plurality of weld points to said second side of said film layer; and wherein said weld points are substantially inelastic and the weld points rupture to form apertures when the laminate is stretched in a cross-machine direction to an elongation of from 50% to 200%. Advantageously this allows to create breathability of the panel upon use i.e. post manufacture. The extent of breathability may be determined by the user by extending more or less of the panel around the wearer's waist. This has the advantage of reduced risk of bacterial contamination within the pores during storage and to improve panel integrity during the production/assembly process (especially when joining the insert to said panel).

The unitary back panel **3** has a substantially trapezoidal shape, preferably an isosceles trapezoidal shape. Such allows for a large stretchable surface providing a better fit, particularly when the panel is the back panel for fitting against the back of the wearer thus providing also additional resistance to leakage. The front panel **2** may have the same or different shape than the unitary back panel **3** and has a total surface area that is smaller than that of said back panel **3.**

The present invention is suitable for open-type diapers, wherein the front and back waist regions may be joined by using fasteners rather than a pant-type seam.

In an embodiment, the article has a front half defined by the edges of the article and a transverse centre line **x-x** of the article, and a rear half defined by the edges of the article and a transverse centre line **x-x** of the article, and a surface area of the front half is preferably within the range 60 to 90%, more preferably within the range 70 to 90%, still more preferably within the range 70 to 80% of a surface area of the rear half, as measured in an extended state of the article. This may provide a more pant-like shape of the absorbent article.

In an embodiment, the insert **4** and/or the liquid-absorbing core **7** extends over the one or more elastified materials (or elastic panels, preferably the back panel) along the longitudinal axis **y-y** such that at least 10%, preferably between 15% and 35%, of the total surface area of said liquid-absorbing core **7** is comprised within the elastified region of the front or back panels. An advantage of this arrangement is to provide a snug fitting core without it bulging out, thus providing a discrete profile that comfortably adheres to the wearer's body.

In an embodiment, the elastic web material used to generate elasticity within the front and/or back panels is arranged to provide an elongation of from 200 to 300%, measured according to the method described herein. It has been found that if the elastic web material has an elongation below 200%, the resulting substrate is too loose which in turn leads to increase risk of leakage, on the other hand if the elongation is above 300%, the substrate becomes too tight and thus limiting freedom of movement and increasing the risk of skin irritation.

In an embodiment, the elastic web material is selected from the group consisting of an elastic film (including laminates of elastic film and nonwoven), elastic fibers, elastic strings, elastic networks, elastic nonwovens, and combinations thereof. Examples of suitable elastic networks are in the form of elastic extruded square netting provided such as those manufactured by Conwed^{®} global netting solutions. Most preferred are elastic nonwovens.

The elastic laminate may comprise first and second layers of fibrous material and an elastic film layer disposed between the first and second fibrous layers, the fibrous layers may be in the form of nonwoven layers. Alternatively, similar elastic laminates may be made by laminating at least two nonwoven layers together, wherein at least one of the two nonwoven layers comprises an elastic nonwoven. This arrangement allows for a more premium feel and brings the subject experience closer to traditional underwear, not only the look, but also the touch and behaviour.

The elastic panel may have a Water Vapour Transmission Rate according to ASTM E96-00 Procedure D of at least 1800 or 1900 g/m², preferably at least 2000 or 2500 g/m², more preferably at least 3000 g/m², even more preferably from 3200 to 5000 g/m², per 24h.

In an embodiment, the portion of the crotch region of the insert **4** between the front and back panels **2,3** comprise one or more nonelastic materials (such as one or more nonwovens as described above), typically arranged such that said region is substantially inelastic (i.e. typically free of elastic web material such as elastic films and the like). An advantage of this arrangement is a reduction in cost in that an elastic material in this particular region can be omitted as it is believed not to negatively impact the comfort and leakage prevention. Moreover, without wishing to be bound by theory it is believed that if this region was elastified (i.e. a continuous elastified surface from front to back waist regions), a greater risk of wrinkle formation would result, such leading to undesirable skin irritation, discomfort, as well as unpleasant aesthetic appearance.

In a preferred embodiment, the front panel(s) and the insert comprise, preferably consist of, inelastic materials. In case the insert comprises elastified cuffs or leg cuffs (as will be described later herein below) bonded to one or more of its surfaces, the presence of these is not excluded by said requirement provided that all remaining layers and components of the insert are inelastic.

In an embodiment, the absorbent core **7** of the insert **4** may comprise superabsorbent polymer (typically in the form of particles) and/or cellulosic fibers (or fluff). Due to the high concentrations of superabsorbent particles, or other high-absorbency material, in the retention portion (i.e. the portion whithin the crotch region exposed to the larges flow of exudates), there can be an increased difficulty with regard to containing the high-absorbency particles within the retention portion and restricting the movement or migration of the superabsorbent onto the bodyside of the pants. To improve the containment of the high-absorbency material, the absorbent structure can include an improved overwrap, such as a wrap sheet, placed immediately adjacent and around the retention portion. The wrap sheet is preferably a layer of absorbent material which covers the major bodyside and outerside surfaces of the retention portion, and preferably encloses substantially all of the peripheral edges of the retention portion to form a substantially complete envelope thereabout. Alternatively, the wrap sheet can provide an absorbent wrap which covers the major bodyside and outerside surfaces of the retention portion, and encloses substantially only the lateral side edges of the retention portion. Accordingly, both the linear and the inwardly curved portions of the lateral side edges of the wrap sheet would be closed about the retention portion. In such an arrangement, however, the end edges of the wrap sheet may not be completely closed around the end edges of the retention portion at the waistband regions of the article. The wrap sheet may comprise a multi-element wrapsheet which includes a separate bodyside wrap layer and a separate outerside wrap layer, each of which extends past all or some of the peripheral edges of the retention portion. Such a configuration of the wrap sheet can, for example, facilitate the formation of a substantially complete sealing and closure around the peripheral edges of the retention portion. The bodyside and outerside layers of the wrap sheet may be composed of substantially the same material, or may be composed of different materials. For example, the outerside layer of the wrap sheet may be composed of a relatively lower basis weight material having a relatively high porosity, such as a wet strength cellulosic tissue composed of softwood pulp. The bodyside layer of the wrap sheet may comprise one of the previously described wrap sheet materials (for example may comprise a meltblown web composed of meltblown polypropylene fibers or low porosity cellulosic tissue web composed of a blend of hardwood/softwood fibers) which has a relatively low porosity. The low porosity bodyside layer can better prevent the migration of superabsorbent particles onto the wearer's skin, and the high porosity, lower basis weight outerside layer can help reduce costs.

Superabsorbent materials suitable for use in the present invention are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 75% by weight.

In an embodiment, the core (or absorbent material) **7** of the insert **4** comprises one or more interconnected channels free of absorbent material such that a cup formation when warn by a subject is formed. Preferably, the insert **4** may further comprise an aqusition distribution layer (ADL) positioned between the topsheet **5** and the core **7.**

In an embodiment, the insert **4** of the absorbent article comprises opposite longitudinal side portions which comprise a pair of elasticized, longitudinally-extending "leg cuffs". The leg cuffs are generally adapted to fit about the legs of a wearer when in use and serve as a mechanical barrier to the lateral flow of body exudates. Leg cuffs are elasticized by leg elastics. The diaper further can comprise a front waist elastic and/or a rear waist elastic. Materials suitable for use in forming leg elastics are known to those skilled in the art. Exemplary of such materials are strands or ribbons of a polymeric, elastomeric material which are adhered to the diaper at the leg cuff while in a stretched position, or which are attached to the diaper while the diaper is pleated, such that elastic constrictive forces are imparted to the leg cuff. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

### THE PROCESS OF MAKING

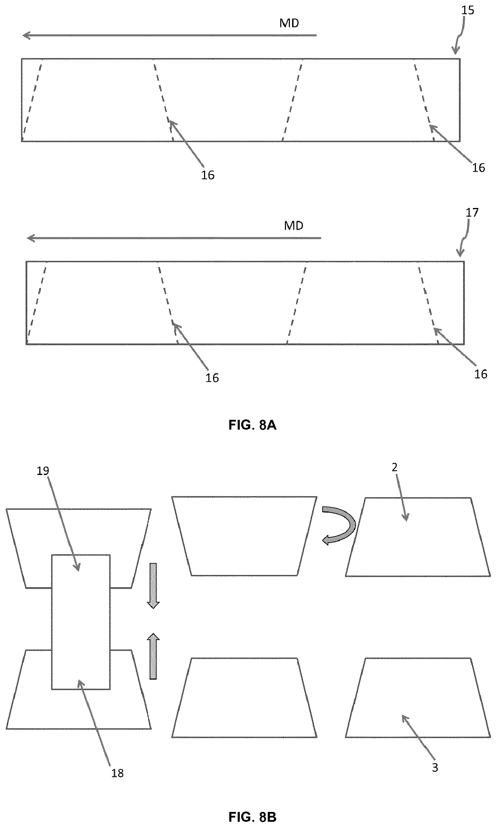

In an embodiment, illustrated in Fig. 8A and 8B, the method of making an absorbent article **1** as described herein comprises the, preferably sequential, steps of: providing a first, preferably inelastic, web **15** having a width extending perpendicular to a machine direction **MD** and a length extending parallel to said machine direction **MD** and accelerating said first web **15** along said machine direction **MD;** severing the first web **15** along cutting lines **16** extending along the entire width thereof, said cutting lines **16** being sequentially applied along said machine direction **MD** and wherein at least each second cutting line **16** in a sequence of cutting lines **16** run substantially parallel to each other, such that a plurality of discrete front panels **2** are formed; providing a second, preferably elastic, web **17** having a width extending perpendicular to a machine direction **MD** and a length extending parallel to said machine direction **MD** and accelerating said second web **17** along said machine direction **MD;** severing the second web **17** along cutting lines **16** extending along the entire width thereof, said cutting lines **16** being sequentially applied along said machine direction **MD** and wherein at least each second cutting line **16** in a sequence of cutting lines **16** run substantially parallel to each other, such that a plurality of discrete back panels **3** are formed; providing a plurality of inserts **4** comprising a liquid permeable topsheet, a liquid impermeable backsheet and an absorbent material sandwiched therebetween; joining only one of said back panels **3** to each said inserts **4** at a first end **18** thereof; joining one or two, preferably only one, of said front panels **2** to each said inserts **4** at a second end **19** thereof being opposite said first end **18** and distanced along the longitudinal axis **y-y;** applying one or more fasteners **8** to the front and/or back panels **2,3;** and wherein only one of the front panel **2** and the back panel **3** is elastic.

Preferably, prior to the step of applying the insert to the front and back panels **2,3,** at least one of the front and back panels **2,3** is rotated by at least 180°, preferably only one of the front and back panels **2,3** is rotated, more preferably only the back panel **3** is rotated.

Preferably wherein the second web **17** consists of an elastic nonwoven or an elastic laminate comprising an elastic film or elastic strands and at least one nonwoven layer adhered thereto; and wherein the first web **15** consists of an inelastic nonwoven layer.

More preferably wherein each front and back panels **2,3** are joined to the garment facing surface of the insert **4** on the backsheet **6** thereof at discrete joining areas **14** that are less than 20%, preferably less than 15%, more preferably from 2% to 10%, even more preferably from 4% to 8%, of the total surface area of said insert **4** taken from a plane parallel to that formed by the longitudinal axis **y-y** and transverse axis **x-x,** and typically have a shape selected from the group consisting of line-form, cross-form, X-shaped, Y-shaped, H-shaped and T-shaped, preferably line-form, more preferably said joining areas comprising an adhesive. The joining step may comprise the step of applying an adhesive in the above mentioned pattern or shape on the front and back panels prior to applying the insert onto the front and back panels or may comprise the step of mechanically bonding the insert to the front and back panels in the above mentioned pattern or shape.

### Method of measuring elongation

This test method is designed to determine the elasticity of elastic web materials in incontinence pants to ensure usability of the pants. The pants are placed in a mounting frame and at predefined forces the percentage of elongation is determined.

**Material:** Tensile tester [like a Zwick / Roell Z1.0] with constant rate of extension calibrated according to the manufacturer's instructions; Clamps and jaws: each jaw face shall be smooth, flat and with a metallic surface and dimension of 30 x 60 mm. The faces shall be parallel and have matching centres with respect to one another in the same clamp and to the corresponding jaw face of the other clamp; Mounting frame: includes a top & bottom device to be placed between the clamps.

**Procedure:** The pants are placed over a mounting frame on the tensile testing machine and pulled with constant rate of extension. Values for elongation of the test products are obtained from a computer interface.

**Definitions:** Elongation: the deformation in the direction of load caused by a tensile force. Elongation is generally expressed as a percentage of the length of the stretched material to the length of the un-stretched material (= distance between frame-ends at the beginning of the test - LE); Extension: the change of length of a material due to stretching.

**Equipment verification:** Verify load cell: a calibrated verification weight of 500g should give a force of 4.90(5) N ± 0.10 N.

**Equipment Parameters:** Following parameters are programmed: Pre-load : 0,1N; Cross Head Speed : 300 mm/min; End of Test: 22N

Gage Length LE (mm) needs to be adjusted depending on the waist size of the pants : Small 220; Medium 250; Large 250; X-Large 270

**Procedure:** Assemble the mounting frame & zero the load cell; Adjust the gage length according to the waist size; Carefully slide the pant over the mounting frame; Zero the load cell; Start the test Force (N) and test path (mm) measurements will be recorded until 22N force is reached; Repeat for at least 5 products.

**Calculation:** Record the elongation value at standard setting forces 2N and 20N for each product. Measure the increase in length from the start of the force-extension curve to the point corresponding with the predefined force. Calculate the elongation as the percentage increase in length based on the gage length for both 2N and 20N.

## Claims

1. An absorbent article (1) comprising:
one or more discrete front panels (2);
a discrete back panel (3); and
an absorbent insert (4) joined to the front and back panels (2,3), wherein said front and back panels (2,3) are separated from each other along a longitudinal axis **y-y** running parallel to a length of said insert (4) and crossing both said front and back panels (2,3), and wherein said insert (4) is interposed between said front panel (2) and said back panel (3) such to form a substantially H-shaped absorbent article, wherein the front and back panels (2,3) have a total width **W** being greater than a width **w** of said insert (4) along a transverse axis **x-x,** and wherein said insert (4) comprises a liquid permeable topsheet (5), a liquid impermeable backsheet (6), and an absorbent material (7) sandwiched between said topsheet (5) and backsheet (6), **characterized in that** the back panel (3) consists of a unitary panel and **in that** the front and/or back panels (2,3) comprise one or more fasteners (8) such that the front panel (2) and back panel (4) can be refastenably coupled together to a closed position onto a wearer, and wherein only one of the front panel (2) and the back panel (3) is elastic, and wherein the unitary back panel (3) has a substantially trapezoidal shape.

2. An absorbent article (1) according to Claim 1 wherein the front panel (2) is a single or unitary panel.

3. An absorbent article (1) according to any of the preceding Claims wherein the front panel (2) is inelastic and the back panel (3) is elastic.

4. An absorbent article (1) according to any of the preceding Claims wherein the insert (4) is joined to the front and back panels (2,3) at discrete joining areas (14), wherein said discrete joining areas are less than 20%, preferably less than 15%, more preferably from 2% to 10%, even more preferably from 4% to 8%, of the total surface area of said insert (4) taken from a plane parallel to that formed by the longitudinal axis **y-y** and transverse axis **x-x.**

5. An absorbent article (1) according to Claim 4 wherein the discrete joining areas have a shape selected from the group consisting of line-form, cross-form, X-shaped, Y-shaped, H-shaped, and T-shaped, preferably line-form.

6. An absorbent article (1) according to any of the preceding Claims wherein the insert (4) is hourglass shaped and comprises a minimum width **wₘᵢₙ** and a maximum width **wₘₐₓ** wherein the ratio **wₘᵢₙ/wₘₐₓ** is from 0.40 to 0.80, preferably from 0.45 to 0.75, more preferably from 0.50 to 0.65.

7. An absorbent article (1) according to any of the preceding Claims wherein the fasteners (8) are arranged to provide a refastenable closure between said front, back panels (2,3), and/or garment facing surface of the insert (4).

8. An absorbent article (1) according to Claim 7 wherein the fasteners (8) are in the form of adhesive and/or mechanical fasteners, and preferably comprise, or consist of, mechanical fasteners in the form of fiber fasteners.

9. An absorbent article (1) according to Claim 8 wherein the fiber fastener comprises a plurality of bonding elements spaced apart from one another, said bonding elements each comprising a head part (9), a backing part (10) and a stem part (11) connecting the head part (9) and the backing part (10) and extending therebetween, and wherein the head part (9) consists of a head plate, wherein the stem part (11) is connected flexibly to the head plate by means of an articulation part (12), and that a contact surface (13) of the head part (9) enables detachable adhesion between the front panel (2), back panel (3) and/or garment facing surface of the insert (4).

10. An absorbent article (1) according to Claim 3 wherein the back panel is activatably breathable.

11. An absorbent article (1) according to any of the preceding Claims wherein the unitary back panel (3) has an isosceles trapezoidal shape.

12. An absorbent article (1) according to Claim 11 wherein the front panel (2) has the same or different shape than the unitary back panel (3) and has a total surface area that is smaller than that of said back panel (3).

13. A method of making an absorbent article (1) according to any of the preceding Claims comprising the steps of:
providing a first web (15) having a width extending perpendicular to a machine direction **MD** and a length extending parallel to said machine direction **MD** and accelerating said first web (15) along said machine direction **MD;**
severing the first web (15) along cutting lines (16) extending along the entire width thereof, said cutting lines (16) being sequentially applied along said machine direction **MD** and wherein at least each second cutting line (16) in a sequence of cutting lines (16) run substantially parallel to each other, such that a plurality of discrete front panels (2) are formed;
providing a second web (17) having a width extending perpendicular to a machine direction **MD** and a length extending parallel to said machine direction **MD** and accelerating said second web (17) along said machine direction **MD;**
severing the second web (17) along cutting lines (16) extending along the entire width thereof, said cutting lines (16) being sequentially applied along said machine direction **MD** and wherein at least each second cutting line (16) in a sequence of cutting lines (16) run substantially parallel to each other, such that a plurality of discrete back panels (3) are formed;
providing a plurality of inserts (4) comprising a liquid permeable topsheet, a liquid impermeable backsheet and an absorbent material sandwiched therebetween;
joining only one of said back panels (3) to each said inserts (4) at a first end (18) thereof;
joining one or two, preferably only one, of said front panels (2) to each said inserts (4) at a second end (19) thereof being opposite said first end (18) and distanced along the longitudinal axis y-y;
applying one or more fasteners (8) to the front and/or back panels (2,3); and wherein only one of the front panel (2) and the back panel (3) is elastic.

14. A method according to Claim 13 wherein the second web (17) consists of an elastic nonwoven or an elastic laminate comprising an elastic film or elastic strands and at least one nonwoven layer adhered thereto; and wherein the first web (15) consists of an inelastic nonwoven layer.

15. A method according to Claims 13 or 14 wherein each front and back panels (2,3) are joined to the garment facing surface of the insert (4) on the backsheet (6) thereof at discrete joining areas (14) that are less than 20%, preferably less than 15%, more preferably from 2% to 10%, even more preferably from 4% to 8%, of the total surface area of said insert (4) taken from a plane parallel to that formed by the longitudinal axis **y-y** and transverse axis **x-x,** and have a shape selected from the group consisting of line-form, cross-form, X-shaped, Y-shaped, H-shaped and T-shaped, preferably line-form.

## Patentansprüche

1. Saugfähiger Artikel (1), umfassend:
eine oder mehrere separate vordere Tafeln (2);
eine separate hintere Tafel (3); und
einen saugfähigen Einsatz (4), der mit der vorderen und hinteren Tafel (2, 3) verbunden ist, wobei die vordere und hintere Tafel (2, 3) voneinander entlang einer Längsachse y-y getrennt sind, die parallel zu einer Länge des Einsatzes (4) verläuft und sowohl die vordere Tafel als auch die hintere Tafel (2, 3) kreuzt, und wobei der Einsatz (4) derart zwischen der vorderen Tafel (2) und der hintern Tafel (3) eingefügt ist, dass er einen im Wesentlichen H-förmigen saugfähigen Artikel bildet, wobei die vordere und hintere Tafel (2, 3) eine gesamte Breite W aufweisen, die grösser als eine Breite w des Einsatzes (4) entlang einer Querachse x-x ist, und wobei der Einsatz (4) eine flüssigkeitsdurchlässige obere Folie (5), eine flüssigkeitsundurchlässige hintere Folie (6) und ein saugfähiges Material (7) umfasst, das zwischen die obere Folie (5) und die hintere Folie (6) eingeschoben ist, **dadurch gekennzeichnet, dass** die hintere Tafel (3) eine einheitliche Tafel umfasst, und dadurch, dass die vordere und/oder hintere Tafel (2, 3) eine oder mehrere Befestigungsvorrichtungen (8) umfassen, so dass die vordere Tafel (2) und die hintere Tafel (4) wiederverschließbar zusammen in eine geschlossene Position auf einem Träger gekoppelt werden können, und wobei nur eine der vorderen Tafel (2) und der hinteren Tafel (3) elastisch ist, wobei die einheitliche hintere Tafel (3) eine im Wesentlichen trapezartige Form aufweist.

2. Saugfähiger Artikel (1) nach Anspruch 1, wobei die vordere Tafel (2) eine einzige oder einheitliche Tafel ist.

3. Saugfähiger Artikel (1) nach einem der vorhergehenden Ansprüche, wobei die vordere Tafel (2) unelastisch und die hinteren Tafel (3) elastisch ist.

4. Saugfähiger Artikel (1) nach einem der vorhergehenden Ansprüche, wobei der Einsatz (4) mit der vorderen und hinteren Tafel (2, 3) an separaten Verbindungsbereichen (14) verbunden ist, wobei die separaten Verbindungsbereiche weniger als 20 %, vorzugsweise weniger als 15 %, insbesondere von 2 % bis 10 %, noch bevorzugter von 4 % bis 8 % des gesamten Flächenbereichs des Einsatzes (4) genommen von einer Ebene ist, die parallel zu derjenigen ist, die durch die Längsachse y-y und Querachse x-x gebildet ist.

5. Saugfähiger Artikel (1) nach Anspruch 4, wobei die separaten Verbindungsbereiche eine Form aufweisen, ausgewählt aus der Gruppe, bestehend aus Linienform, Kreuzform, X-Form, Y-Form, H-Form und T-Form, vorzugsweise Linienform.

6. Saugfähiger Artikel (1) nach einem der vorhergehenden Ansprüche, wobei der Einsatz (4) sanduhrförmig ist und eine minimale Breite wₘᵢₙ und eine maximale Breite wₘₐₓ umfasst, wobei das Verhältnis wₘᵢₙ/wₘₐₓ von 0,40 bis 0,80, vorzugsweise von 0,45 bis 0,75, insbesondere von 0,50 bis 0,65 ist.

7. Saugfähiger Artikel (1) nach einem der vorhergehenden Ansprüche, wobei die Befestigungsvorrichtungen (8) angeordnet sind, um einen wiederverschließbaren Verschluss zwischen der vorderen, der hinteren Tafel (2, 3) und/oder der auf die Kleidung gerichteten Fläche des Einsatzes (4) bereitzustellen.

8. Saugfähiger Artikel (1) nach Anspruch 7, wobei die Befestigungsvorrichtungen (8) die Form von haftenden und/oder mechanischen Befestigungsvorrichtungen aufweisen und vorzugsweise mechanische Befestigungsvorrichtungen in Form von Faserbefestigungsvorrichtungen umfassen oder daraus bestehen.

9. Saugfähiger Artikel (1) nach Anspruch 8, wobei die Faserbefestigungsvorrichtung eine Vielzahl von Bindungselementen umfasst, die voneinander beabstandet sind, wobei die Bindungselemente jeweils einen Kopfteil (9), einen Stützteil (10) und einen Schaftteil (11) umfassen, der den Kopfteil (9) und den Stützteil (10) verbindet und sich dazwischen erstreckt, und wobei der Kopfteil (9) aus einer Kopftafel besteht, wobei der Schaftteil (11) flexibel mit der Kopftafel mit Hilfe eines Gelenkteils (12) verbunden ist, und eine Kontaktfläche (13) des Kopfteils (9) eine abnehmbare Haftung zwischen der vorderen Tafel (2), der hinteren Tafel (3) und/oder der auf die Kleidung gerichteten Fläche des Einsatzes (4) ermöglicht.

10. Saugfähiger Artikel (1) nach Anspruch 3, wobei die hintere Tafel aktivierbar atmungsaktiv ist.

11. Saugfähiger Artikel (1) nach einem der vorhergehenden Ansprüche, wobei die einheitliche hintere Tafel (3) eine gleichschenklige trapezartige Form aufweist.

12. Saugfähiger Artikel (1) nach Anspruch 11, wobei die vordere Tafel (2) die gleiche oder eine verschiedene Form als die einheitliche hintere Tafel (3) aufweist und einen gesamten Oberflächenbereich aufweist, der kleiner als derjenige des Stützteils (3) ist.

13. Verfahren zum Herstellen eines saugfähigen Artikels (1) nach einem der vorhergehenden Ansprüche, umfassend die folgenden Schritte:
Bereitstellen einer ersten Bahn (15) mit einer Breite, die sich senkrecht zu einer Maschinenrichtung MD erstreckt, und einer Länge, die sich parallel zu der Maschinenrichtung MD erstreckt und die erste Bahn (15) entlang der Maschinenrichtung MD beschleunigt;
Trennen der ersten Bahn (15) entlang Schnittlinien (16), die sich entlang der gesamten Breite davon erstrecken, wobei die Schnittlinien (16) aufeinanderfolgend entlang der Maschinenrichtung MD aufgebracht werden, und wobei mindestens jede zweite Schnittlinie (16) in einer Aufeinanderfolge von Schnittlinien (16) im Wesentlichen parallel zueinander verlaufen, so dass eine Vielzahl von separaten vorderen Tafeln (2) gebildet wird;
Bereitstellen eines zweiten Netzes (17) mit einer Breite, die sich senkrecht zu einer Maschinenrichtung MD erstreckt, und einer Länge, die sich parallel zu der Maschinenrichtung MD erstreckt und die zweite Bahn (17) entlang der Maschinenrichtung MD beschleunigt;
Trennen der zweiten Bahn (17) entlang Schnittlinien (16), die sich entlang der gesamten Breite davon erstrecken, wobei die Schnittlinien (16) aufeinanderfolgend entlang der Maschinenrichtung MD aufgebracht werden, und wobei mindestens jede zweite Schnittlinie (16) in einer Aufeinanderfolge von Schnittlinien (16) im Wesentlichen parallel zueinander verlaufen, so dass eine Vielzahl von separaten hinteren Tafeln (3) gebildet wird;
Bereitstellen einer Vielzahl von Einsätzen (4), umfassend eine flüssigkeitsdurchlässige obere Folie, eine flüssigkeitsundurchlässige hintere Folie und ein saugfähiges Material, das dazwischen eingeschoben ist;
Verbinden nur einer der hinteren Tafeln (3) mit jedem der Einsätze (4) an einem ersten Ende (18) davon;
Verbinden einer oder zwei, vorzugsweise nur einer, der vorderen Tafeln (2) mit jedem der Einsätze (4) an einem zweiten Ende (19) davon, das gegenüber dem ersten Ende (18) und beabstandet entlang der Längsachse y-y ist;
Aufbringen einer oder mehrerer Befestigungsvorrichtungen (8) auf die vordere und/oder hintere Tafel (2, 3), und wobei nur eine der vorderen Tafel (2) und der hinteren Tafel (3) elastisch ist.

14. Verfahren nach Anspruch 13, wobei die zweite Bahn (17) aus einem elastischen Vliesstoff oder einem elastischen Laminat besteht, umfassend einen elastischen Film oder elastische Stränge und mindestens eine Vilesstoffschicht, die daran gehaftet ist; und wobei die erste Bahn (15) aus einer nicht elastischen Vliesstoffschicht besteht.

15. Verfahren nach Anspruch 13 oder 14, wobei jede vordere und hintere Tafel (2, 3) mit der auf die Kleidung gerichteten Fläche des Einsatzes (4) auf der hinteren Folie (6) davon an separaten Verbindungsbereichen (14) verbunden sind, die weniger als 20 %, vorzugsweise weniger als 15 %, insbesondere von 2 % bis 10 %, noch bevorzugter von 4 % bis 8 % des gesamten Flächenbereichs des Einsatzes (4) genommen von einer Ebene ist, die parallel zu derjenigen ist, die durch die Längsachse y-y und Querachse x-x gebildet ist, und eine Form aufweisen, ausgewählt aus der Gruppe, bestehend aus Linienform, Kreuzform, X-Form, Y-Form, H-Form und T-Form, vorzugsweise Linienform.

## Revendications

1. Article absorbant (1) comprenant :
un ou plusieurs panneau(x) avant discret(s) (2) ;
un panneau arrière discret (3) ; et
un insert absorbant (4) joint aux panneaux avant et arrière (2, 3), dans lequel lesdits panneaux avant et arrière (2, 3) sont séparés l'un de l'autre le long d'un axe longitudinal y-y parallèle à une longueur dudit insert (4) et qui croise lesdits panneaux avant et arrière (2, 3), et dans lequel ledit insert (4) est interposé entre ledit panneau avant (2) et ledit panneau arrière (3) afin de former un article absorbant sensiblement en forme de H, dans lequel les panneaux avant et arrière (2, 3) possèdent une largeur totale W qui est supérieure à une largeur w dudit insert (4) le long d'un axe transversal x-x, et dans lequel ledit insert (4) comprend une couche de finition perméable au liquide (5), une couche de support imperméable au liquide (6), et un matériau absorbant (7) pris en sandwich entre ladite couche de finition (5) et la couche de support (6), **caractérisé en ce que** le panneau arrière (3) se compose d'un panneau unitaire et **en ce que** les panneaux avant et/ou arrière (2, 3) comprennent une ou plusieurs fixation(s) (8) de sorte que le panneau avant (2) et le panneau arrière (4) puissent être couplés ensemble de manière re-fixable en une position fermée sur une personne qui les porte, et dans lequel un seul du panneau avant (2) et du panneau arrière (3) est élastique, et dans lequel le panneau arrière unitaire (3) possède une forme sensiblement trapézoïdale.

2. Article absorbant (1) selon la revendication 1, dans lequel le panneau avant (2) est un panneau simple ou unitaire.

3. Article absorbant (1) selon l'une quelconque des revendications précédentes, dans lequel le panneau avant (2) est inélastique et le panneau arrière (3) est élastique.

4. Article absorbant (1) selon l'une quelconque des revendications précédentes, dans lequel l'insert (4) est joint aux panneaux avant et arrière (2, 3) au niveau de zones de jonction discrètes (14), dans lequel lesdites zones de jonction discrètes représentent moins de 20%, de préférence moins de 15%, de préférence 2% à 10%, de préférence 4% à 8%, de la surface totale dudit insert (4) prise depuis un plan parallèle à celui formé par l'axe longitudinal y-y et l'axe transversal x-x.

5. Article absorbant (1) selon la revendication 4, dans lequel les zones de jonction discrètes possèdent une forme choisie parmi le groupe consistant en une forme de ligne, une forme de croix, une forme de X, une forme de Y, une forme de H, et une forme de T, de préférence une forme de ligne.

6. Article absorbant (1) selon l'une quelconque des revendications précédentes, dans lequel l'insert (4) est en forme de sablier et comprend une largeur minimum wₘᵢₙ et une largeur maximum wₘₐₓ, dans lequel le rapport wₘᵢₙ/wₘₐₓ est compris entre 0,40 et 0,80, de préférence entre 0,45 et 0,75, de préférence entre 0,50 et 0,65.

7. Article absorbant (1) selon l'une quelconque des revendications précédentes, dans lequel les fixations (8) sont prévues pour offrir une fermeture re-fixable entre lesdits panneaux avant, arrière (2, 3), et/ou une surface de l'insert (4) tournée vers un vêtement.

8. Article absorbant (1) selon la revendication 7, dans lequel les fixations (8) sont sous la forme de fixations adhésives et/ou mécaniques, et comprennent de préférence, ou se composent de, des fixations mécaniques sous la forme de fixations à fibres.

9. Article absorbant (1) selon la revendication 8, dans lequel la fixation à fibres comprend une pluralité d'éléments de liaison espacés les uns des autres, lesdits éléments de liaison comprenant chacun une partie de tête (9), une partie d'appui (10) et une partie de tige (11) qui relie la partie de tête (9) et la partie d'appui (10) et qui s'étend entre elles, et dans lequel la partie de tête (9) se compose d'une plaque de tête, dans lequel la partie de tige (11) est reliée de manière flexible à la plaque de tête à l'aide d'une partie d'articulation (12), et une surface de contact (13) de la partie de tête (9) permet une adhésion détachable entre le panneau avant (2), le panneau arrière (3) et/ou la surface tournée vers un vêtement de l'insert (4).

10. Article absorbant (1) selon la revendication 3, dans lequel le panneau arrière est respirable de manière activable.

11. Article absorbant (1) selon l'une quelconque des revendications précédentes, dans lequel le panneau arrière unitaire (3) possède une forme trapézoïdale isocèle.

12. Article absorbant (1) selon la revendication 11, dans lequel le panneau avant (2) possède la même forme ou une forme différente de celle du panneau arrière unitaire (3) et possède une surface totale inférieure à celle dudit panneau arrière (3).

13. Procédé de fabrication d'un article absorbant (1) selon l'une quelconque des revendications précédentes, comprenant les étapes consistant à :
prévoir une première bande (15) qui possède une largeur qui s'étend perpendiculairement à un sens machine MD et une longueur qui s'étend parallèlement audit sens machine MD et qui fait accélérer ladite première bande (15) le long dudit sens machine MD ;
séparer la première bande (15) le long de lignes de découpe (16) qui s'étendent sur la totalité de sa longueur, lesdites lignes de découpe (16) étant appliquées successivement le long dudit sens machine MD, et dans lequel au moins chaque seconde ligne de découpe (16) d'une séquence de lignes de découpe (16) est sensiblement parallèle aux autres, de sorte que plusieurs panneaux avant discrets (2) soient formés ;
prévoir une seconde bande (17) qui possède une largeur qui s'étend perpendiculairement à un sens machine MD et une longueur qui s'étend parallèlement audit sens machine MD et qui fait accélérer ladite seconde bande (17) le long dudit sens machine MD ;
séparer la seconde bande (17) le long de lignes de découpe (16) qui s'étendent sur la totalité de sa largeur, lesdites lignes de découpe (16) étant successivement appliquées le long dudit sens machine MD, et dans lequel au moins chaque seconde ligne de découpe (16) d'une séquence de lignes de découpe (16) est sensiblement parallèle aux autres, de sorte que plusieurs panneaux arrière discrets (3) soient formés ;
prévoir une pluralité d'inserts (4) comprenant une couche de finition perméable au liquide, une couche d'appui imperméable au liquide et un matériau absorbant pris en sandwich entre elles ;
joindre un seul desdits panneaux arrière (3) à chacun desdits inserts (4) à une première extrémité (18) de ceux-ci ;
joindre un ou deux, de préférence un seul, desdits panneaux avant (2) à chacun desdits inserts (4) à une seconde extrémité (19) de ceux-ci opposée à ladite première extrémité (18) et distancée le long de l'axe longitudinal y-y ;
appliquer une ou plusieurs fixation(s) (8) sur les panneaux avant et/ou arrière (2, 3) ; et dans lequel un seul du panneau avant (2) et du panneau arrière (3) est élastique.

14. Procédé selon la revendication 13, dans lequel la seconde bande (17) se compose d'un stratifié non tissé élastique ou d'un stratifié élastique comprenant un film élastique ou des brins élastiques et au moins une couche de non-tissé qui adhère à celui-ci/ceux-ci ; et dans lequel la première bande (15) se compose d'une couche de non-tissé élastique.

15. Procédé selon la revendication 13 ou 14, dans lequel chaque panneau avant et arrière (2, 3) est joint à la surface tournée vers un vêtement de l'insert (4) sur la couche d'appui (6) de celui-ci au niveau de zones de jonction discrètes (14) qui représentent moins de 20%, de préférence moins de 15%, de préférence entre 2% et 10%, de préférence entre 4% et 8% de la surface totale dudit insert (4) prise depuis un plan parallèle à celui formé par l'axe longitudinal y-y et l'axe transversal x-x, et possède une forme choisie parmi le groupe consistant en une forme de ligne, une forme de croix, une forme de X, une forme de Y, une forme de H et une forme de T, de préférence une forme de ligne.
